(19) Europäisches Patentamt
European Patent Office
Office européen des brevets

(11) Publication number: **0 051 234**
**B1**

(12) **EUROPEAN PATENT SPECIFICATION**

(45) Date of publication of patent specification: **13.03.85**

(21) Application number: **81108875.6**

(22) Date of filing: **24.10.81**

(51) Int. Cl.$^4$: **C 07 D 205/08,**
C 07 D 471/04,
C 07 D 498/04,
C 07 D 487/04,
C 07 D 499/00, C 07 D 501/02

# (54) A process for the production of a 2-azetidinone derivative.

(30) Priority: **31.10.80 JP 152151/80**

(43) Date of publication of application:
**12.05.82 Bulletin 82/19**

(45) Publication of the grant of the patent:
**13.03.85 Bulletin 85/11**

(84) Designated Contracting States:
**AT BE CH DE FR GB IT LI NL**

(56) References cited:
**EP-A-0 020 883**
**US-A-4 193 917**

**TETRAHEDRON LETTERS, 1970, 1901 c, J. ORG. CHEM., 45, 1135, 1142 (1980)**

(73) Proprietor: **ZAIDAN HOJIN BISEIBUTSU KAGAKU KENKYU KAI**
**14-23, Kami Osaki 3-chome**
**Shinagawa-ku Tokyo (JP)**

(72) Inventor: **Ohno, Masaji**
**1565-13, Koshigoe**
**Kamakura-shi Kanagawa-ken (JP)**
Inventor: **Umezawa, Hamao**
**23, Toyotama Kita 4-chome**
**Nerima-ku Tokyo (JP)**

(74) Representative: **Meyer-Dulheuer, Karl-Hermann, Dr. et al**
**HOECHST Aktiengesellschaft Zentrale**
**Patentabteilung Postfach 80 03 20**
**D-6230 Frankfurt/Main 80 (DE)**

**EP 0 051 234 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

BACKGROUND OF THE INVENTION

Field of the invention

This invention relates to a new and efficient process for the production of a 2-azetidinone derivative which is useful as a starting compound for the synthesis of thienamycin, nocardicin and other β-lactam group antibiotics.

2-Azetidinone is a compound which is forming the main skeleton of β-lactam group antibiotics, and the β-lactam group antibiotics have gained great attention in the recent years amongst many kinds of antibacterial compounds. For example, it is well known that the 2-azetidinone skeleton of the formula

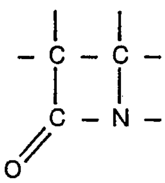

exists in penicillin group antibiotics and cephalosporin group antibiotics which now occupy the premier position as the antibacterial antibiotics, as well as in carbapenem antibiotics such as thienamycin and nocardicin which are called the fourth-generation antibiotics.

For the synthesis of the compounds containing the 2-azetidinone skeleton, namely the 2-azetidinone derivatives, there have been proposed several methods, for example, the method of producing 2-azetidinone from β-alanine by Grignard reaction (J. Am. Chem. Soc., *71*, 2124 (1949); Chem. & Ind., *1964*, 2097), the method of producing 2-azetidinone by condensing an N-substituted β-alanine in the presence of thionyl chloride and a tertiary amine (J. Org. Chem., *23*, 1102 (1958)), and the method of producing a 2-azetidinone derivative by treating a β-amino acid derivative with dicyclohexylcarbodiimide (Tetrahedron Letters, *21*, 2783 (1980); J. Am. Chem. Soc., *102*, 2060 (1980)). However, these methods previously proposed can hardly be said to be the commercially available processes, because they suffer from the drawbacks that the feasibility of the process depends on the chemical structure of the compound used as the starting material, that expensive reagents are needed, and/or that the yield of the desired product is poor.

In this situation, it is highly desirable to provide a new process for the preparation of β-lactam ring in an easy way starting from commercially inexpensive initial materials and reagents, and this eventually makes it feasible to achieve the total synthesis of the β-lactam antibiotics such as thienamycin, nocardicin and olivanic acid which have gained great attention in the recent years. Such a new process will obviously have a great value in commerce.

Thus, we have carried out extensive research for new processes for converting a β-amino acid compound into a β-lactam compound, that is, a 2-azetidinone derivative, and as a result we have now found that when the β-amino acid compound is treated with a system comprising triphenylphosphine and a heterocyclic disulfide in the presence of an alkylnitrile, and the β-amino acid compound employed can undergo intramolecular condensation it is readily converted into the corresponding 2-azetidinone derivative in a favorable yield. On the basis of this finding, we have completed this invention.

According to this invention, therefor, there is provided a process for the production of a 2-azetidinone derivative of the formula

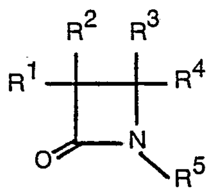

(I)

wherein $R^1$ and $R^2$ each denote a hydrogen atom, an amino group, a substituted amino group, an alkoxyl group or a mono-valent hydrocarbon residue, unsubstituted or substituted with an inert substituent, $R^3$ and $R^4$ each denote a hydrogen atom or a mono-valent hydrocarbon residue, unsubstituted or substituted with an inert substituent, $R^5$ denotes a hydrogen atom or a mono-valent hydrocarbon residue, unsubstituted or substituted with an inert substituent, or $R^4$ and $R^5$ taken together form a ring which remains as the residue of a β-lactam group antibiotic when the moiety of β-lactam ring is removed from said β-lactam group antibiotic, the process comprising reacting a β-amino acid compound of the formula

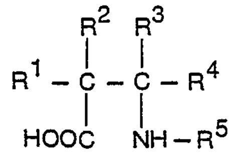

(II)

wherein $R^1$, $R^2$, $R^3$, $R^4$ and $R^5$ are each as defined above, with triphenylphosphine and a heterocyclic disulfide of the formula

$$R^6—S—S—R^6 \qquad (III)$$

wherein $R^6$ is a heterocyclic group selected from pyridyl, benzothiazolyl, benzimidazolyl and pyrimidyl, in the presence as solvent of an alkylnitrile of the formula

$$R^7CN \qquad (IV)$$

wherein $R^7$ is an alkyl group, particularly an alkyl group of 1 to 6 carbon atoms, at a temperature of from ambient temperature to the boiling temperature of the reaction mixture.

In the β-amino acid compound of the formula (II), $R^1$ and/or $R^2$ may denote(s) a substituted amino group which may, for example, be an aralkylamino group such as benzylamino, and triphenylmethylamino, or an aralkyloxy- or an alkyloxy-carbonylamino group such as benzyloxycarbonylamino, methoxycarbonylamino, ethoxycarbonylamino, *tert*-butoxycarbonylamino and tert-amyloxycarbonylamino, an alkanoylamino group such as acetylamino and propionylamino, an aralkanoylamino group such as phenylacetylamino, phenoxyacetylamino, α-hydroxyphenylacetylamino or α-sulfophenylacetylamino, a heteroalkanoylamino group such as 2-thienylacetylamino, 1-(1H)-tetrazolylacetylamino, (4-pyridylthio)acetylamino or (2-amino-4-thiazolyl)acetylamino, a cyanoacetylamino group, or an α-methoxyimino-2-furylacetylamino group. Again $R^1$ and/or $R^2$ may denote an alkoxyl group which may, for example, a lower alkoxyl group of 1 to 6 carbon atoms such as methoxyl, ethoxyl, n- or iso-propoxyl, n-, iso-, sec- or tert-butoxyl.

The groups $R^1$, $R^2$, $R^3$, $R^4$ and $R^5$ independently may represent a mono-valent hydrocarbon residue (which may also be termed as a hydrocarbyl group) which may be linear or cyclic (either aromatic or aliphatic) or a combination of these and which may preferably contain 10 or less carbon atoms, in general. The hydrocarbon residue is desirably a saturated one. Suitable examples of the mono-valent hydrocarbon residue include an alkyl group, particularly a lower alkyl group such as methyl, ethyl, n- or iso-propyl, n-, iso-, sec- or tert-butyl, an alkenyl group such as allyl, an alkynyl group such as ethynyl, a cycloalkyl group of 3 to 7 carbon atoms such as cyclohexyl and cycloheptyl, an aryl group such as phenyl and naphthyl, and an aralkyl group such as benzyl, phenethyl and phenylpropyl.

The mono-valent hydrocarbon residue just mentioned may optionally be substituted with an inert substituent, and as this inert substituent there may be mentioned, for example, a hydroxyl group, a lower alkoxycarbonyl group, an alkoxyl group, an alkylthio group, an acyloxy group, particularly an alkanoyloxy group such as acetoxy or an aroyloxy group such as benzoyloxy, and a carbonyl group.

Thus, the mono-valent hydrocarbon residue, unsubstituted or substituted with an inert substituent, which may be present for the groups $R^1$ to $R^5$ in the starting β-amino acid compound of the formula (II) and hence in the 2-azetidinone compound of the formula (I) includes, for example, 1-hydroxyethyl, 2-hydroxyethyl, hydroxymethyl, 1-hydroxy-2-methylethyl, 1-hydroxy-3-phenylpropyl, acetyl, propionyl, 3-oxobutyl, methoxycarbonylmethyl, methoxycarbonylethyl, ethoxycarbonylmethyl, tert-butoxycarbonylmethyl, methoxyethyl, acetoxyethyl, 2-acetoxypropyl, tetrahydropyranyloxyethyl groups and the like.

In some cases, the groups $R^4$ and $R^5$ taken together may form a ring which remains as the residue of a β-lactam group antibiotic when the β-lactam ring is removed from said β-lactam group antibiotic. Such ring may, for example, be of the following formulae:—

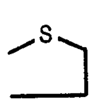 , 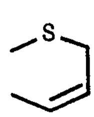 , 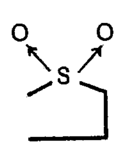 , 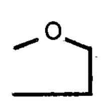 , 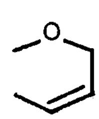 , 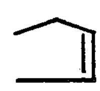 , 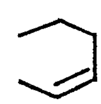 .

By the term "lower" herein used is meant that the group or compound to which the term "lower" is attached contains up to 6 carbon atoms, preferably up to 4 carbon atoms.

Typical examples of the β-amino acid compound of the formula (II) used as the starting material in the process of this invention include β-alanine, α-methoxy-β-alanine, α-amino-β-alanine, α-(1-hydroxyethyl)-β-alanine, α-ethyl-β-alanine, α-amino-β-(2-hydroxyethyl)-β-alanine, β-(2-hydroxyethyl)-β-alanine, β-(2-acetoxyethyl)-β-alanine, β-phenyl-β-alanine, N-benzyl-β-alanine, β-aminoglutaric acid mono-methyl ester, β-aminoglutaric acid mono-ethyl ester and β-aminoglutaric acid mono-tert-butyl ester.

The process of this invention may be shown by the following equation:—

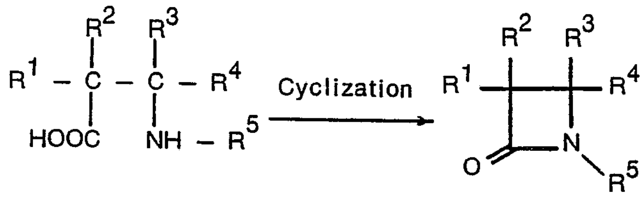


(II) (I)

In the process of this invention, the cyclization of the starting β-amino acid compound (II) is effected using triphenylphosphine and a heterocyclic disulfide as the condensation agent in the presence of an alkylnitrile as described hereinbefore. The heterocyclic disulfides which are used in combination with triphenylphosphine as the condensation agent for the above-mentioned cyclization reaction in the present process may, for example, be 2,2'-dipyridyl disulfide, 2,2'-benzothiazolyl disulfide, 2,2'-dibenzimidazolyl disulfide and 4,4'-dipyridyl disulfide, 2,2'-dipyrimidyl disulfide and so on.

The quantities of the triphenylphosphine and heterocyclic disulfide used as the condensation agent are not critical but may vary in a wide range depending on the nature of the starting compound used, the reaction conditions employed and the other factors of operating the process. In general, however, both of the triphenylphosphine and heterocyclic disulfide may be at least 1.0 molar proportion and preferably 1.0 to 1.3 molar proportions for 1.0 molar proportion of the starting β-amino acid compound used. The triphenylphosphine and heterocyclic disulfide may usually be used in equimolar proportions.

In the process of this invention, the cyclization reaction using the condensation agent comprising the combination of the triphenylphosphine and heterocyclic disulfide is readily carried out in a manner known per se as described in, e.g. the "Tetrahedron Letters" *1970*, 1901.

In accordance with this invention, the cyclization reaction is found to proceed very smoothly and very efficiently at a neutral pH value when it is conducted in the presence of an alkylnitrile. The alkylnitrile available for this purpose includes, for example, acetonitrile and propionitrile. Acetonitrile is most preferred. The quantity of the alkylnitrile used is not critical and may vary over a wide range depending on the nature of the starting β-amino acid compound used, the reaction conditions employed and the other factors of operating the process. It is preferable that the alkylnitrile is used in excess so that the alkylnitrile itself can serve also as the solvent of the reaction.

The reaction temperature at which the above cyclization of the β-amino acid compound is effected according to this invention is not critical but may vary widely usually in the range from ambient temperature to the boiling temperature of the reaction mixture. It is preferred to carry out the reaction at the refluxing temperature of the reaction mixture. The reaction may proceed under atmospheric, sub-atmospheric or superatmospheric pressure.

It has been found that the reaction of cyclizing the β-amino acid compound with formation of the β-lactam ring can be affected by the concentration of the starting β-amino acid compound present in the reaction system, and that the yield of the desired 2-azetidinone derivative is likely to decrease with increased concentration of the starting β-amino acid compound. Advantageously, the β-amino acid compound may be present in the reaction system generally at a concentration of about 0.001 M to about 1 M and preferably of about 0.01 to about 0.1 M. Accordingly, when the process of this invention is carried out, it is possible to add the starting β-amino acid compound or triphenylphosphine or the disulfide dissolved in a solvent dropwise to the reaction mixture so that the concentration of the β-amino acid compound in the reaction system is controlled to a preferred level.

To recover the desired 2-azetidinone compound of the formula (I) from the reaction mixture after the completion of the reaction and to purify the product, it is possible to follow a known recovery and purification technique, for example, extraction with organic solvent, chromatography and recrystallization.

The process of this invention is able to afford the 2-azetidinone derivative of the formula (I) in a favorably high yield, and this desired product is very useful as an intermediate for synthesis of a variety of β-lactam group antibiotics. For instance, according to the process of this invention, a 4-lower alkoxycarbonylmethyl-2-azetidinone of the formula (I-a) shown below can be prepared from a β-aminoglutaric acid mono-lower alkyl ester. From the 4-lower alkoxycarbonylmethyl-2-azetidinone can be synthetized thienamycin via the route as described in the "J. Org. Chem., *45*, 1130, 1135, 1142 (1980) and as shown by the following equation:—

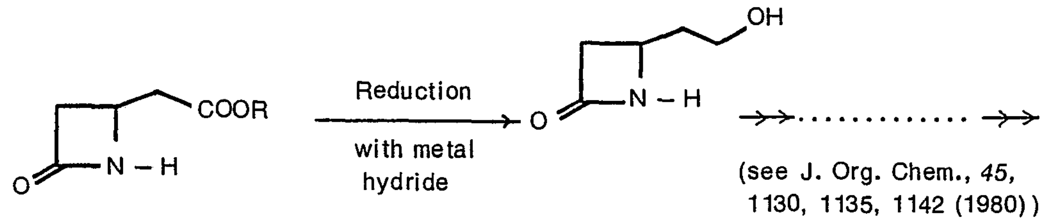


(I–a)

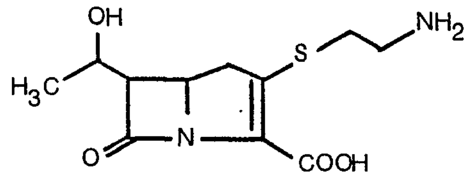

Thienamycin

Most of the β-amino acid compounds of the formula (II) which are used as the starting compound in the process of this invention are known, but some of them are new compounds. For instance, such compounds of the formula (II) where $R^1$, $R^2$, $R^3$ and $R^5$ are all a hydrogen atom and $R^4$ is a lower alkoxycarbonylmethyl group, that is, the compound of the formula

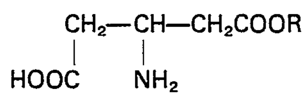
(II-a)

wherein R denotes a lower alkyl group is a novel compound which has never been described in the literature. The new compound of the formula (II-a) may be prepared by a new process comprising reacting a β-N-protected or unprotected aminoglutaric acid di-alkyl ester of the formula

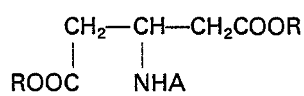
(V)

wherein R is a lower alkyl group and A is a hydrogen atom or an amino-protecting group removable by hydrogenolysis or mild hydrolysis, with an ester-hydrolyzing enzyme (an esterase) in an organic solvent at a pH and at a temperature at which the enzyme employed is enzymatically active, to remove hydrolytically and preferentially one ester-forming alkyl group (R) from the compound (V) without cleavage of the other ester-forming alkyl group, and then removing, if necessary, the residual amino-protecting group (A) from the resulting hydrolysis product (see Japanese patent application Nos. 146,344/80 and 146,345/80). In this new process, the amino group of the compound (V) may be protected with an amino-protecting group such as a benzyloxycarbonyl group. When this new process is conducted using a β-aminoglutaric acid di-(lower)alkyl ester of which the amino group is unprotected, there is afforded an optically active β-(R)-aminoglutaric acid mono-(lower)alkyl ester (the half ester). While, when this new process is conducted using the β-N-protected aminoglutaric acid di-(lower)alkyl ester of which the amino group has been protected e.g. with a benzyloxycarbonyl group, there is afforded an optically active β-(S)-aminoglutaric acid mono-(lower)alkyl ester (the half ester).

When the β-aminoglutaric acid mono-alkyl ester of the above formula (II-a) so prepared is cyclized by applying thereto the process of this invention, there is obtained a 2-azetidinone compound of the formula

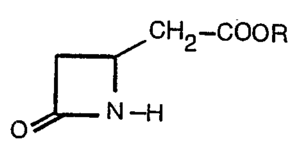
(I–a)

wherein R is as defined above, which is a novel compound, and which, depending on the steric configuration of the compound (II-a) employed, has the (R)- or (S)-configuration as shown below.

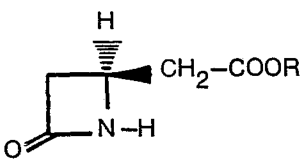

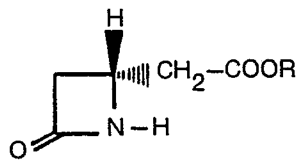

(I—a—1) (I—a—2)

Among them, the (S)-isomer of the formula (I-a-I) is especially useful as it can be utilized for the production of thienamycin via the synthetic route as stated hereinbefore.

This invention is illustrated with reference to the following Examples.

Example 1

A suspension of 165 mg (1.02 milimol) of mono-methyl β-(S)-aminoglutarate in 20 ml of acetonitrile was admixed with 315 mg (1.20 milimol) of triphenylphosphine and 264 mg (1.20 milimol) of dipyridyl disulfide, and the mixture was heated at 55—60°C for 12 hours under stirring. As the reaction proceeded, the reaction solution turned yellow.

After the reaction was completed, the reaction solution was concentrated under a reduced pressure and the residue was chromatographed on a column of 40 g of silica gel and eluted with ethyl ether-methylene chloride (1:1 by volume). The eluate was collected in 10 ml-fractions, and the fraction Nos. 10 to 20 containing the desired product was again chromatographed on a column of 20 g of silica gel in the same manner as above. The fractions of the eluate containing the desired product were combined together and concentrated to dryness in vacuo to afford 123 mg of the desired 4-(S)-methoxy-carbonylmethyl-2-azetidinone.

Yield 84%.

The infrared absorption spectrum, nuclear magnetic resonance spectrum, mass spectrometry and optical rotation of the above product are given below.

IR spectrum (KBr): 3420, 1765 (β-lactam), 1740 $cm^{-1}$. NMR. spectrum: 2.4—.29 (m, 3H, $\underline{CH_2}COOCH_3$ & $C_3$—H), 3.16 (d d d(J=2, 5, 15 Hz), 1H, $C_3$—H), 3.72 (s, 3H, $COO\underline{CH_3}$), 3.95 (m, 1H, $C_4$—H), 6.15 (br, 1H, N—H).

Mass spectrometry: $M^+ + 1 = 144$

Optical rotation: $[\alpha]^{25}_D = +65.34°$ (c 1.11, $CHCl_3$).

The mono-methyl β-(S)-aminoglutarate which was employed as the starting compound in the process of Example 1 as above was prepared by the following procedures.

(a) To a solution of 4.68 g (26.7 milimol) of dimethyl β-aminoglutarate in 40 ml of dioxane were added dropwise a solution of 5.47 g (32.1 milimol) of benzyloxycarbonyl chloride in 20 ml of dioxane and a solution of 3.25 g (32.1 milimol) of triethylamine in 20 ml of dioxane. The mixture obtained was stirred at ambient temperature for 2 hours, and the crystals deposited were removed by filtration. The filtrate was concentrated and the residue was purified by chromatography on a silica gel column developed with ethyl ether-methylene chloride (1:1), yielding 7.41 g (90%) of dimethyl β-benzyloxycarbonylaminoglutarate as an oil.

(b) A solution of 465 mg (1.5 milimol) of dimethyl β-benzyloxycarbonylaminoglutarate in 1.5 ml of acetone was admixed with 45 ml of a phosphate buffer solution (pH 8.0). The solution obtained was admixed with 2.7 mg (measured as the quantity of protein) in pig liver esterase (Esterase E—3128, a product of Shigma Co., U.S.A.). The resultant admixture was allowed to stand at 25°C for 6 hours under occasional stirring, and the resulting reaction solution was adjusted to pH 1 by addition of 2.5 ml of 2N hydrochloric acid thereto, followed by extraction with 50 ml of methylene chloride. The organic layer was dried over anhydrous sodium sulfate and concentrated to give 419 mg of a crude product as crystals. This crude product was purified by chromatography on silica gel developed with ethyl ether to afford 410 mg (93%) of optically active mono-methyl β-(S)-benzyloxycarbonylaminoglutarate which showed a specific optical rotation $[\alpha]^{25}_D = +0.71°$ (c 7.45, $CHCl_3$). mp. 97.0—97.5°C.

IR spectrum (KBr): 3330, 2840—2960, 1740, 1725, 1700 $cm^{-1}$.

H—NMR. spectrum ($CDCl_3$): δ2.69 (bd, 4H, $\underline{CH_2}$ J=6 Hz), δ3.65 (s, 2H, $CO_2\underline{Me}$), δ4.17—4.53 (m, 1H, $\underline{CH}$), δ5.07 (s, 2H, $\underline{CH_2Ph}$), δ5.63 (bd, 1H, $\underline{NH}$), δ7.30 (s, 5H, Ph).

(c) The mono-methyl β-(S)-benzyloxycarbonylaminoglutarate (385 mg, 1.3 milimol) obtained as above was dissolved in 20 ml of methanol, and the resulting methanolic solution was admixed with 40 mg of 10% palladium-carbon and then stirred at ambient temperature for 30 minutes under atmosphere of hydrogen. The reaction solution was filtered and the filtrate solution was concentrated to give 205 mg (98%) of mono-methyl β-(S)-aminoglutarate as oil which showed a specific optical rotation $[\alpha]^{25}_D = -5.52°$ (c 3.26, $H_2O$).

This product showed the following IR and NMR spectra and elemental analysis.

IR. spectrum: 3440, 2400—2900, 1730 $cm^{-1}$.

$^1$H—NMR. spectrum ($D_2O$): δ2.59 (bd, 2H, J=6 Hz), δ2.84 (bd, 2H, J=6 Hz), δ3.76 (s, 3H, $CO_2\underline{CH_3}$), δ3.85—4.05 (m, 1H, $\underline{CH}$).

Example 2

Mono-methyl β-(R)-aminoglutarate (57 mg, 0.35 milimol), triphenylphosphine (110 mg, 0.42 milimol) and dipyridyl disulfide (93 mg, 0.42 milimol) were reacted with each other in 4 ml of acetonitrile in the same manner as in Example 1, and the resulting reaction mixture was processed in the same manner as in Example 1 for purification by chromatography on a column of 15 g of silica gel. The desired 4-(R)-methoxycarbonylmethyl-2-azetidinone was obtained in a yield of 37 mg (73%). This product showed the following properties.

IR. spectrum (KBr): 3420, 1765 (β-lactam), 1740 $cm^{-1}$. NMR. spectrum: 2.4—2.9 (m, 3H, $\underline{CH_2}COOCH_3$ & $C_3$—H), 3.16 (1H, $C_3$—H), 3.72 (s, 3H, —$COO\underline{CH_3}$), 3.95 (m, 1H, $C_4$—H), 6.15 (br, 1H, N—H).

Optical rotation: $[\alpha]_D^{25} = -26.03°$ (c 1.26 $CHCl_3$).

Mass spectrometry: $M^+ + 1 = 144$

The mono-methyl β-(R)-aminoglutarate which was employed as the starting compound in the process of Example 2 above was prepared by the following procedure.

A solution of dimethyl β-aminoglutarate (346 mg, 2.0 milimol) in 7 ml of a phosphate buffer solution (pH 8.0) was admixed with 2.89 mg (400 units) of pig liver esterase (Esterase E—3128, a product of Shigma Co., U.S.A.). The resultant admixture was allowed to stand at 25°C for 2.5 hours under occasional stirring, and the reaction solution was adjusted to a pH of about 7.0 by addition of 0.1 N hydrochloric acid, followed by concentration to dryness at a temperature of not higher than 20°C. The oily residue was extracted with 50 ml of methanol, and the methanolic extract was concentrated to give 268 mg of a crude product. This crude product was isolated and purified by chromatography on cellulose column (Cellulose CF—11, a product of Wattman Co., U.S.A.) developed with n-butanol saturated with water as the eluent, to afford 212 mg (66% yield) of monomethyl β-(R)-aminoglutarate as oil which showed a specific optical rotation $[\alpha]_D^{25} = +2.36°$ (c 4.23, $H_2O$).

IR. spectrum (KBr): 3440, 1730, 2400—2900 $cm^{-1}$. NMR. ($D_2O$): δ2.59 (bd, 2H, J=6 Hz), δ2.84 (bd, J=6 Hz), δ3.76 (s, 3H, $COO\underline{CH_3}$), δ3.85—4.05 (m, 1H, $\underline{CH}$).

Example 3

To a solution of 157.4 mg (0.6 milimol) of triphenylphosphine and 132.2 mg (0.6 milimol) of dipyridyl disulfide in 50 ml of acetonitrile was added 82.6 mg (0.5 milimol) of β-phenyl-β-alanine, and the resultant admixture was refluxed for 4.5 hours under stirring to effect the intermolecular condensation. After the reaction was completed, the reaction solution was concentrated to dryness under reduced pressure and the residue was chromatographed on a column of 40 g of silica gel developed with ethyl ether-methylene chloride (1:1 by volume). The eluate was collected in 10 ml-fractions, and the fractions containing the desired product were subjected to a preparative thin layer chromatography on a column of silica gel developed with ethyl ether-benzene (2:1 by volume) for the purification purpose. The desired 4-phenyl-2-azetidinone was obtained in a yield of 67.2 mg (91%). This product had the following properties.

IR. spectrum: 3220, 1740, 1710 $cm^{-1}$. NMR. spectrum: δ2.82 (ddd, (J=14.9, 2.8, 0.9 Hz), 1H, $C_3$—H), δ3.40 (ddd, (J=14.9, 5.2, 2.3 Hz), 1H, $C_3$—H), δ4.69 (dd, (J=5.2, 2.6 Hz), 1H, $C_4$—H), δ6.66 (br, 1H, N—H), δ7.32 (s, 5H, $C_6H_5$).

Example 4—10

The starting β-amino acid compound indicated in the table below was reacted with triphenylphosphine and 2,2′-dipyridyl disulfide in acetonitrile in a manner similar to the process of Examples 1 to 3 under the reaction conditions indicated in the table below. The 2-azetidinone compounds indicated in the table below were obtained in a yield indicated below.

For comparative purpose, β-phenyl-β-alanine was reacted with triphenylphosphine and dipyridyl disulfide in the absence of acetonitrile (as the typical example of the alkylnitrile to be used according to this invention) but in the presence of dimethylformimide (DMF) or nitromethane as the reaction medium, and the results obtained were shown in the table below.

TABLE 1

| Example | Starting β-amino acid compound | Reaction Conditions: 2-Azetidinone product | Solvent | Initial Concentration (M) of starting compound | Reaction temperature (°C) | Reaction time (hrs) | Yield (%) |
|---|---|---|---|---|---|---|---|
| 4 | 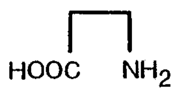 | 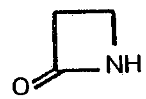 | $CH_3CN$ | 0.1<br>0.01 | 55<br>under reflux | 24<br>4.5 | 39<br>60 |
| 5 | 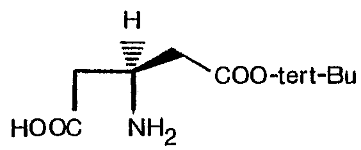 | 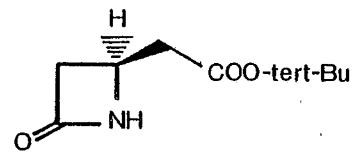 | $CH_3CN$ | 0.01 | under reflux | 12 | 57 |
| 6 | 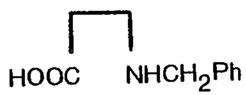 | 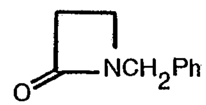 | $CH_3CN$ | 0.1<br>0.1<br>0.01 | 60<br>under reflux<br>under reflux | 6<br>4.5<br>4.5 | 36<br>44<br>91 |
| 7 | 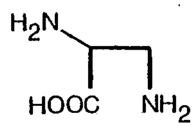 | 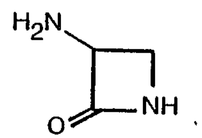 | $CH_3CN$ | 0.1<br>0.01 | under reflux<br>under reflux | 4.5<br>4.5 | 24<br>54 |
| 8—10 | 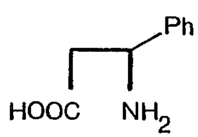 | 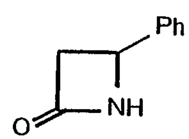 | $CH_3CN$ | 0.1<br>0.01 | 55<br>under reflux | 4.5<br>4.5 | |
| | | | DMF (Comparative) | 0.1<br>0.01 | 55<br>under reflux | 4.5<br>4.5 | 9<br>12 |
| | | | $CH_3NO_2$ (Comparative) | 0.1<br>0.01 | under reflux<br>under reflux | 4.5<br>4.5 | 8<br>< 26 |

**Claims**

1. A process for the production of a 2-azetidinone derivative of the formula

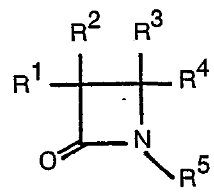

(I)

wherein $R^1$ and $R^2$ each denote a hydrogen atom, an amino group, a substituted amino group, an alkoxyl group or a mono-valent hydrocarbon residue, unsubstituted or substituted with an inert substituent, $R^3$ and $R^4$ each denote a hydrogen atom or a mono-valent hydrocarbon residue, unsubstituted or substituted with an inert substituent, $R^5$ denotes a hydrogen atom or a mono-valent hydrocarbon residue, unsubstituted or substituted with an inert substituent, or $R^4$ and $R^5$ taken together form a ring which remains as the residue of a β-lactam group antibiotic when the β-lactam ring is removed from said β-lactam group antibiotic, the process comprising reacting a β-amino acid compound of the formula

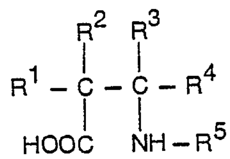

(II)

wherein $R^1$, $R^2$, $R^3$, $R^4$ and $R^5$ are each as defined above, with triphenylphosphine and a heterocyclic disulfide of the formula

$$R^6—S—S—R^6 \qquad (III)$$

wherein $R^6$ is a heterocyclic group selected from pyridyl, benzothiazolyl, benzimidazolyl and pyrimidyl, in the presence as solvent of an alkylnitrile of the formula

$$R^7CN \qquad (IV)$$

wherein $R^7$ is an alkyl group, particularly an alkyl group of 1 to 6 carbon atoms, at a temperature of from ambient temperature to the boiling temperature of the reaction mixture at a neutral pH value.

2. A process of Claim 1 in which the alkylnitrile used is acetonitrile or propionitrile.

3. A process of Claim 1 or 2 in which the heterocyclic disulfide used is dipyridyl disulfide.

4. A process of Claim 1 or 2 in which triphenylphosphine and the heterocyclic disulfide are used in equimolar or substantially equimolar proportions with respect to each other.

5. A process of Claim 1 or 2 in which the β-amino acid compound used is present at a concentration of 0.001 M to 1 M in the reaction system.

6. The compound of the formula

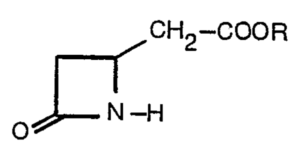

(I–a)

wherein R is an alkyl group containing 1 to 6 carbon atoms.

**Revendications**

1. Procédé de préparation de dérivés de la 2-azétidinone de formule

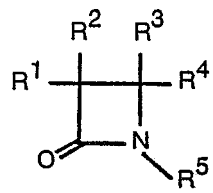

(I)

dans laquelle $R^1$ et $R^2$ représentent chacun un atome d'hydrogène, un groupe amino, un groupe amino substitué, un groupe alcoxy ou un radial hydrocarbure monovalent, non-substitué ou substitué avec un substituant inerte, $R^3$ et $R^4$ représentent chacun un atome d'hydrogène ou un radical hydrocarbure monovalent, non-substitué ou substitué avec un substituant inerte, $R^5$ représente un atome d'hydrogène ou un radical hydrocarbure monovalent, non-substitué ou substitué avec un substituant inerte, ou $R^4$ et $R^5$ forment ensemble un noyau qui demeure comme reste d'un antibiotique du groupe du β-lactame lorsque le noyau β-lactame est éliminé de l'antibiotique du groupe du β-lactame, le procédé consistant à faire réagir un composé d'acide β-aminé de formule

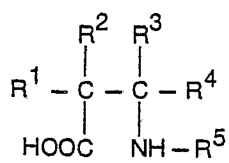

(II)

dans laquelle $R^1$, $R^2$, $R^3$, $R^4$ et $R^5$ sont chacun tels que définis ci-dessus, avec de la triphénylphosphine et un disulfure hétérocyclique de formule

$$R^6—S—S—R^6 \qquad (III)$$

dans laquelle $R^6$ est un groupe hétérocyclique choisi parmi les groupes pyridyle, benzothiazolyle, benzimidazolyle et pyrimidyle, en présence, comme solvant, d'un nitrile alkylique de formule

$$R^7CN \qquad (IV)$$

dans laquelle $R^7$ est un groupe alkyle, en particulier un groupe alkyle ayant de 1 à 6 atomes de carbone, à une température comprise entre la température ambiante et la température d'ébullition du mélange de réaction, à un pH neutre.

2. Procédé selon la revendication 1, dans lequel le nitrile alkylique utilisé est l'acétonitrile ou le propionitrile.

3. Procédé selon l'une des revendications 1 et 2, dans lequel le disulfure hétérocyclique est le disulfure de dipyridyle.

4. Procédé selon l'une des revendications 1 et 2, dans lequel la triphénylphosphine et le disulfure hétérocyclique sont utilisés en des proportions équimolaires ou pratiquement équimolaires, l'une par rapport à l'autre.

5. Procédé selon l'une des revendication 1 et 2, dans lequel le composé d'acide β-aminé utilisé est présent à une concentration de 0,001 à 1 M dans le système réactionnel.

6. Composé de formule

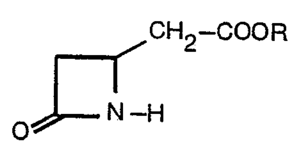

dans laquelle R est un groupe alkyle ayant de 1 à 6 atomes de carbone.

**Patentansprüche:**

1. Verfahren zur Herstellung von 2-Azetidinonderivaten der Formel

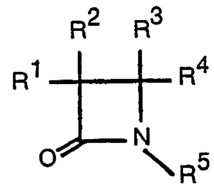

(I)

worin $R^1$ und $R^2$ je für ein Wasserstoffatom, eine gegebenenfalls substituierte Aminogruppe, eine Alkoxygruppe oder einen gegebenenfalls mit einem inerten Substituenten substituierten einwertigen Kohlenwasserstoffrest, $R^3$ und $R^4$ je für ein Wasserstoffatom oder einen gegebenenfalls mit einem inerten Substituenten substituierten einwertigen Kohlenwasserstoffrest und $R^5$ für ein Wasserstoffatom oder einen gegebenenfalls mit einem inerten Substituenten substituierten einwertigen Kohlenwasserstoffrest stehen oder $R^4$ und $R^5$ zusammen einen Ring bilden, der bei Wegnahme des β-Lactamrings aus einem Antibiotikum mit β-Lactamgruppe als Rest verbleibt, dadurch gekennzeichnet, dass man eine β-Aminosäureverbindung der Formel

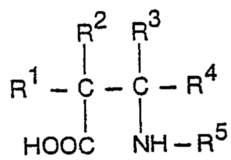

(II)

worin $R^1$, $R^2$, $R^3$, $R^4$ und $R^5$ jeweils die oben angegebenen Bedeutungen haben, bei einer Temperatur von Raumtemperatur bis zum Siedepunkt des Reaktionsgemisch und bei neutralem pH-Wert mit Triphenylphosphin und einem heterocyclischen Disulfid der Formel

$$R^6—S—S—R^6 \qquad (III)$$

worin $R^6$ eine unter Pyridyl, Benzothiazolyl, Benzimidazolyl und Pyrimidyl ausgewählte heterocyclische Gruppe darstellt, in Gegenwart eines Alkylnitrils der Formel

$$R^7CN \qquad (IV)$$

worin $R^7$ eine Alkylgruppe mit insbesondere 1 bis 6 Kohlenstoffatomen darstellt, als Lösungsmittel umsetzt.

2. Verfahren nach Anspruch 1, dadurch gekennzeichnet, dass man als Alkylnitril Acetonitril oder Propionitril einsetzt.

3. Verfahren nach Anspruch 1 oder 2, dadurch gekennzeichnet, dass man als heterocyclisches Disulfid Dipyridyldisulfid einsetzt.

4. Verfahren nach Anspruch 1 oder 2, dadurch gekennzeichnet, dass man Triphenylphosphin und das heterocyclische Disulfid in äquimolaren oder weitgehend äquimolaren Anteilen zueinander einsetzt.

5. Verfahren nach Anspruch 1 oder 2, dadurch gekennzeichnet, dass die verwendete β-Aminosäureverbindung im Reaktionssystem in einer Konzentration von 0,001 m bis 1 m vorliegt.

6. Verbindung der Formel

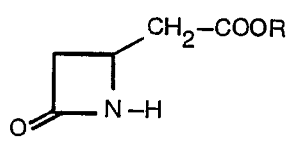

worin R eine Alkylgruppe mit 1 bis 6 Kohlenstoffatomen darstellt.